# EUROPEAN PATENT APPLICATION

(11) **EP 3 228 617 A1**
(43) Date of publication of application: **11.10.2017**
(21) Application number: 17164739.9
(22) Date of filing: 04.04.2017
(51) Int. Cl.: C07D 309/06, C09K 19/34, C07D 309/04

(54) **PRODUCTION METHOD FOR TETRAHYDRO-2H-PYRAN DERIVATIVE**

(30) Priority: 08.04.2016 JP 2016077955; 27.10.2016 JP 2016210895
(71) Applicant: JNC Corporation, Chiyoda-ku Tokyo 100-8105 (JP); JNC Petrochemical Corporation, Tokyo 100-0004 (JP)
(72) Inventor: Hirata, Kenji, Chiba, 290-8551 (JP)
(74) Representative: Hoffmann Eitle

(57) **Abstract**

Provided is a production method for a tetrahydro-2H-pyran derivative suitable for industrial production, and an intermediate capable of efficiently synthesizing the derivative.

The tetrahydro-2H-pyran-derivative is synthesized by applying compound (A), compound (B) or compound (C) as an intermediate.

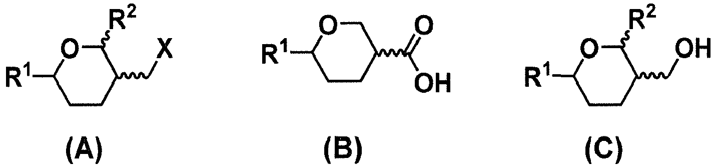

In compound (A), compound (B) and compound (C), R¹ is alkyl, alkenyl or alkoxy, R² is hydrogen or alkoxy, and X is a leaving group.

## Description

### Technical Field

The invention relates to a production method for a tetrahydro-2H-pyran derivative, and an intermediate and a derivative thereof.

### Background Art

Patent literature No. 3 discloses a liquid crystal compound being a tetrahydro-2H-pyran derivative. Then, 5-((4-ethoxy-2,3-difluorophenoxy)methyl)-2-propyltetrahydro -2H-pyran or the like disclosed in Patent literature No.1 is useful as a liquid crystal compound having negative dielectric anisotropy. The synthesis method for the compound is described in Patent literature No. 2 or the like, but requires a comparatively expensive starting material, and has a disadvantage of a large number of steps or a low yield. Therefore, a desire has been expressed for a production method suitable for industrial production.

### Citation List

### Patent Literature

Patent literature No. 1: WO 2008-114821 A.
Patent literature No. 2: WO 2009-136534 A.
Patent literature No. 3: WO 2011-021534 A. **Non-Patent literature**

Non-Patent literature No. 1: Chemische Berichte (1955), 88, 1676-84.
Non-Patent literature No. 2: Chemische Berichte (1959), 92, 877-883.
Non-Patent literature No. 3: Chemische Berichte (1959), 92, 884-894.

### Summary of Invention

### Technical Problem

A first object of the invention is to solve the above-described problems of conventional arts, and to provide a production method for a tetrahydro-2H-pyran derivative suitable for industrial production, and to provide an intermediate capable of efficiently synthesizing a derivative. A second object is to provide a liquid crystal compound, a liquid crystal composition and a liquid crystal display device at a lower cost.

### Solution to Problem

The present inventors have found that a tetrahydro-2H-pyran derivative can be effectively obtained by using compound (A), compound (B) or compound (C), and have completed the invention.

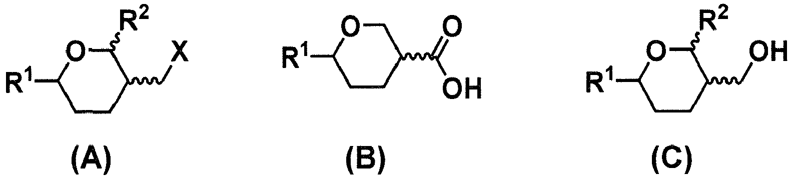

In compound (A), compound (B) and compound (C), R¹ is alkyl, alkenyl or alkoxy, R² is hydrogen or alkoxy, and X is a leaving group.

### Advantageous Effects of Invention

A first advantage of the invention is a capability of providing a production method for a tetrahydro-2H-pyran derivative suitable for industrial production, and a capability of providing an intermediate capable of efficiently synthesizing a derivative. A second advantage is a capability of providing a liquid crystal compound, a liquid crystal composition and a liquid crystal display device at a lower cost.

### Description of Embodiments

One example of synthesis method for compound (A), compound (B) and compound (C) will be described. Non-Patent literature Nos. 1 to 3 describe a synthesis method to compound (12) or compound (13). Synthesis procedures are described in a section in Examples. With regard to a reaction of forming a carbon-carbon bond and a reaction of conversion of a functional group, a method described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.) can be applied, in addition to the methods described in Examples. As a solvent to be used for synthesis, a suitable solvent by which the reaction is not adversary affected can be used, in addition to the solvents described in Examples.

The invention includes items described below.
Item 1. A production method for a tetrahydro-2H-pyran derivative, applying compound (A), compound (B) or compound (C) as an intermediate: 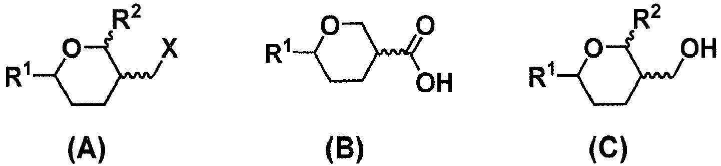 wherein, in compound (A), compound (B) and compound (C), R¹ is alkyl, alkenyl or alkoxy, R² is hydrogen or alkoxy, and X is a leaving group.
Item 2. The production method for tetrahydro-2H-pyran according to item 1, wherein, in compound (A), compound (B) or compound (C), R¹ is alkyl, X is nonafluorosulfonate, trifluoromethanesulfonate, fluorosulfonate, tosilate, mesilate, iodine, bromine or chlorine, and R² is hydrogen.
Item 3. The production method for the tetrahydro-2H-pyran derivative according to item 1 or 2, wherein compound (A), compound (B) or compound (C) that may be substituted and derived from 5-valerolactone is applied as an intermediate.
Item 4. The production method for the tetrahydro-2H-pyran derivative according to any one of items 1 to 3, wherein a compound represented by formula (1) is applied as an objective substance: 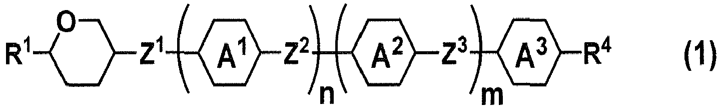 wherein, in formula (1),
   R¹ is alkyl having 1 to 10 carbons;
   R⁴ is hydrogen, cycloalkane having 3 to 8 carbons or alkyl having 1 to 10 carbons, and in the alkyl, at least one piece of -CH₂- may be replaced by -O-, and at least one piece of -CH₂CH₂- may be replaced by -CH=CH-;
   ring A¹, ring A² and ring A³ are independently 1,4-cyclohexylene, 1,4-cyclohexenylene, 1,4-phenylene, 1,4-phenylene in which at least one piece of hydrogen is replaced by halogen, naphthalene-2,5-diyl, naphthalene-2,5-diyl in which at least one piece of hydrogen is replaced by halogen, tetrahydro-2H-pyran-2,5-diyl, pyrimidine-2,5-diyl, pyridine-2,5-diyl or 1,3-dioxane-2,5-diyl;
   Z¹ is -CH₂CH₂-, -CH=CH-, -CH₂O- or -COO-;
   Z² and Z³ are independently a single bond, -CH₂CH₂-, -C=C-, -CH=CH-, -CF₂O-, -OCF₂-, -OCH₂-, -CH₂O-, -OCO-, -COO-, -CH₂CH₂CH₂O- or -OCH₂CH₂CH₂-; and
   n and m are independently 0 or 1.
Item 5. The production method for the tetrahydro-2H-pyran derivative according to any one of items 1 to 4, including a step of an etherification reaction.
Item 6. A compound represented by formula (2): 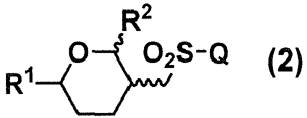 wherein, in formula (2), R¹ is alkyl, alkenyl or alkoxy, and R² is hydrogen or alkoxy; and
   Q is any one of structures described below. 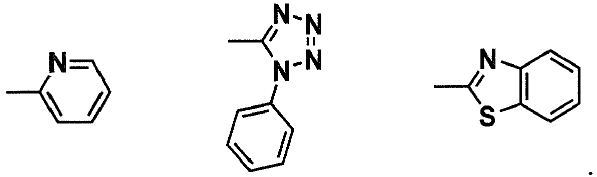
Item 7. Utilization of the compound that is represented by formula (1) and produced by the production method according to item 4 or 5 as a component of a liquid crystal medium.

Then, if substituted δ-valerolactone (10) is allowed to react with sodium hydride and ethyl formate, compound (11) is obtained. If compound (11) is allowed to react with hydrochloric acid in an alcohol solvent, a mixture of compound (12) and compound (13) is obtained. Compound (12) and compound (13) can be separated by silica gel chromatography or the like.

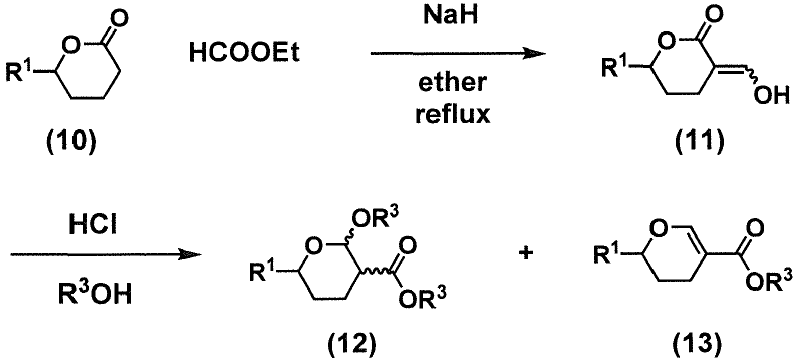

Specific preferred examples of R¹ include alkyl having 1 to 10 carbons, alkenyl having 1 to 10 carbons and alkoxy having 1 to 9 carbons. Further preferred examples include alkyl having 1 to 10 carbons. Still further preferred examples include alkyl having 1 to 5 carbons.

R³ is alkyl. Specific preferred examples of R³ include methyl, ethyl, propyl, isopropyl, butyl and t- butyl. Further preferred examples include methyl and ethyl. Still further preferred examples include methyl.

If compound (12) is subjected to elimination of R³OH in the presence of an acid catalyst, compound (13) is obtained.

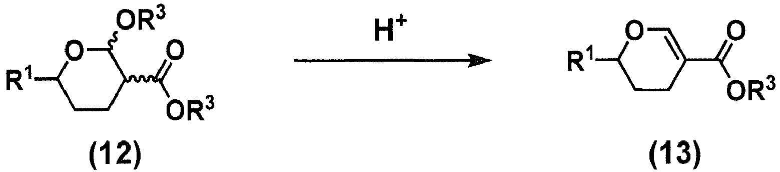

Specific preferred examples of an acid catalyst include hydrochloric acid, sulfuric acid, p-toluenesulfonic acid monohydrate, heteropolyacid and a sulfonic acid-modified silica gel.

If compound (12) is subjected to hydride reduction, compound (14) is obtained.

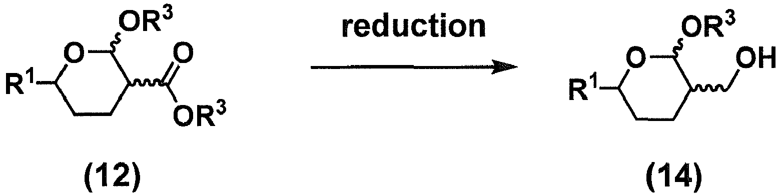

Specific preferred examples of a reducing agent include lithium aluminum hydride, sodium borohydride, diisobutylaluminum hydride and sodium bis(2-methoxyethoxy)aluminum hydride. Further preferred examples include lithium aluminum hydride and sodium bis(2-methoxyethoxy)aluminum hydride.

If compound (13) is subjected to hydrogenation, compound (15) is obtained.

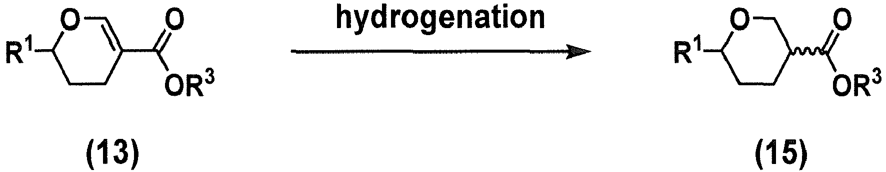

Specific preferred examples of a reduction method include catalytic hydrogenation and silane reduction. From a viewpoint of stereoselectivity, the silane reduction is further preferred.

In the catalytic hydrogenation, specific preferred examples of a catalyst include a supported catalyst such as palladium on carbon and platinum on carbon, palladium black and Raney nickel. As a support, alumina, silica, zeolite, hydroxyapatite or the like may be used in addition to carbon.

In the silane reduction, specific preferred examples of a silane reducing agent include triethylsilane, diphenylsilane, phenylsilane and 1,1,3,3-tetramethyldisiloxane. Further preferred examples include triethylsilane and 1,1,3,3-tetramethyldisiloxane.

In the silane reduction, specific preferred examples of an acid include methanesulfonic acid, trifluoroacetic acid, aluminum chloride, zinc chloride, a boron trifluoride-diethylether complex. Further preferred examples include methanesulfonic acid.

In the silane reduction, a preferred reaction temperature is 50°C or lower. A further preferred temperature is in the range from 0°C to 40°C.

If compound (15) is subjected to hydrolysis, compound (B) is obtained.

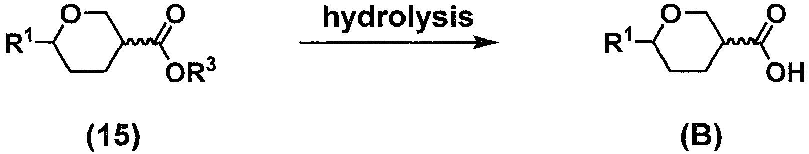

Hydrolysis is preferably performed under basic conditions. Specific preferred examples of a base include potassium hydroxide, sodium hydroxide, lithium hydroxide and calcium hydroxide.

If compound (15) is subjected to hydride reduction, compound (16) is obtained.

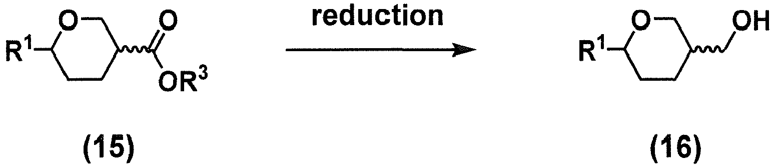

Specific preferred examples a reducing agent include lithium aluminum hydride, sodium borohydride, diisobutylaluminum hydride, sodium bis(2-methoxyethoxy)aluminum hydride and triethylsilane. Further preferred examples include lithium aluminum hydride and sodium bis(2-methoxyethoxy)aluminum hydride.

If a hydrogenation catalyst or a silane reducing agent capable of reducing both olefin and ester is used, compound (16) is obtained from compound (13).

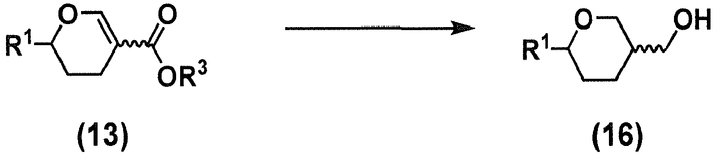

In the catalytic hydrogenation, specific preferred examples of the hydrogenation catalyst include platinum oxide and rhodium oxide.

In the silane reduction, specific preferred examples of the silane reducing agent include triethylsilane, diphenylsilane and 1,1,3,3-tetramethyldisiloxane. Further preferred examples include triethylsilane and 1,1,3,3-tetramethyldisiloxane.

In the silane reduction, specific preferred examples of an acid include methanesulfonic acid, trifluoroacetic acid, aluminum chloride, zinc chloride and a boron trifluoride-diethylether complex.

In the silane reduction, a preferred reaction temperature is 120°C or lower. A further preferred temperature is in the range from 40°C to 100°C.

If compound (13) is subjected to hydride reduction, and then hydrogenation, compound (16) is obtained.

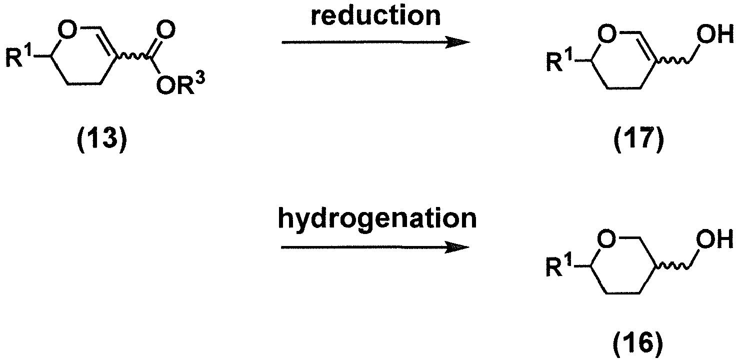

Compound (17) is a dihydropyran derivative, and functions as a protecting group of a hydroxy group under acidic conditions. In consideration of stability of compound (17), the process is preferred in which compound (15) is synthesized from compound (13), and then compound (16) is derived from compound (15).

If compound (B) is subjected to reduction, compound (16) is obtained.

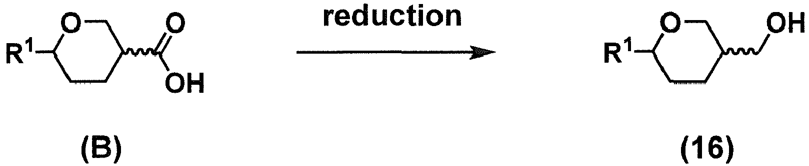

A preferred reducing agent is a borane complex.

The hydroxy group in compound (14) or compound (16) can be converted into a leaving group by a publicly known method.

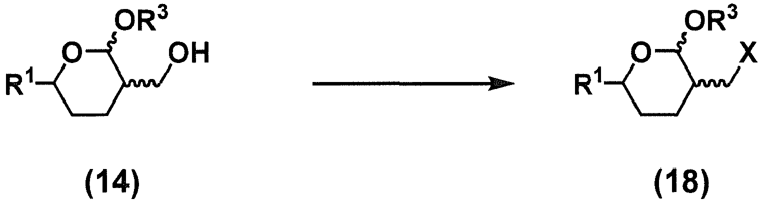

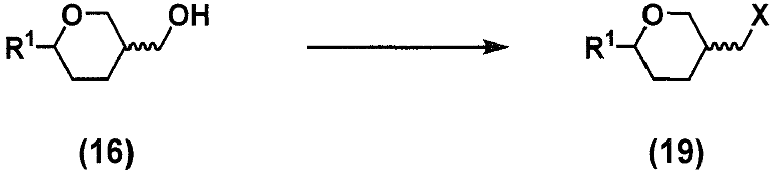

In compound (14) and compound (18), R³ is alkyl. R³ is alkyl, and therefore OR³ serves as alkoxy.

In compound (18) or compound (19), specific preferred examples of X being the leaving group include nonafluorosulfonate, trifluoromethanesulfonate, fluorosulfonate, tosilate, mesilate, iodine, bromine and chlorine. Further preferred examples include tosilate, mesilate, iodine, bromine and chlorine.

Compound (14) and compound (16) correspond to compound (C). Compound (18) and compound (19) correspond to compound (A). Compound (A) can be synthesized with a high yield without using an expensive reagent. From compound (A), phosphonium salt (20) to be used in a Wittig reaction, and sulfone compound (21), compound (22) or compound (23) to be used each in a Julia-Kocienski reaction can be synthesized.

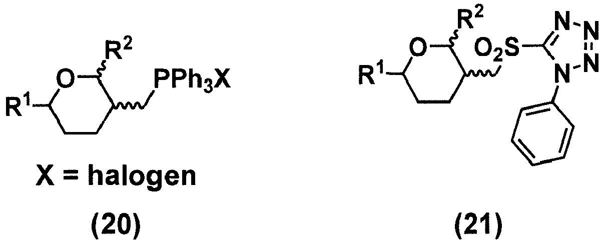

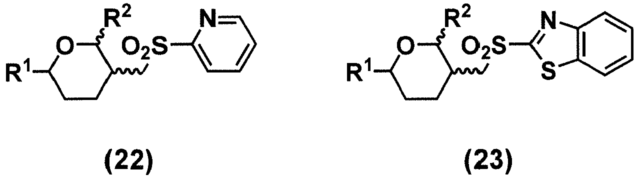

The tetrahydro-2H-pyran derivative can be synthesized by performing an etherification reaction, an esterification reaction and a carbon-carbon formation reaction by using compound (A), compound (B), compound (C), compound (20), compound (21), compound (22) or compound (23).

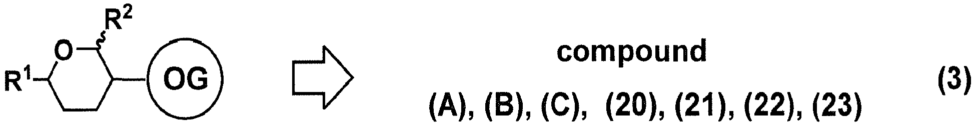

In formula (3), OG is an organic group.

Tetrahydro-2H-pyran has a cis form and a trans form. The trans form is thermodynamically more stable, and is preferred. In a liquid crystal compound used in a liquid crystal display device, the trans form is preferred from a viewpoint of liquid crystallinity.

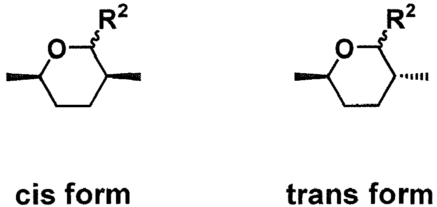

A ratio of the trans form in compound (15), compound (16) or compound (19) is determined by the reduction method for olefin in compound (13). When the silane reduction is performed under preferred conditions, the ratio of the trans form in compound (19) is about 75 to 90%. A trans-tetrahydro-2H-pyran derivative can be further easily synthesized in comparison with conventional arts.

In the etherification reaction, the elimination reaction takes precedence in the cis form in compound (19), and the etherification reaction takes precedence in the trans form. As a result, the ratio of the trans form in the compound is improved. Further, an amount of alcohol, phenol or the like can be reduced to an amount corresponding to the trans form in compound (19).

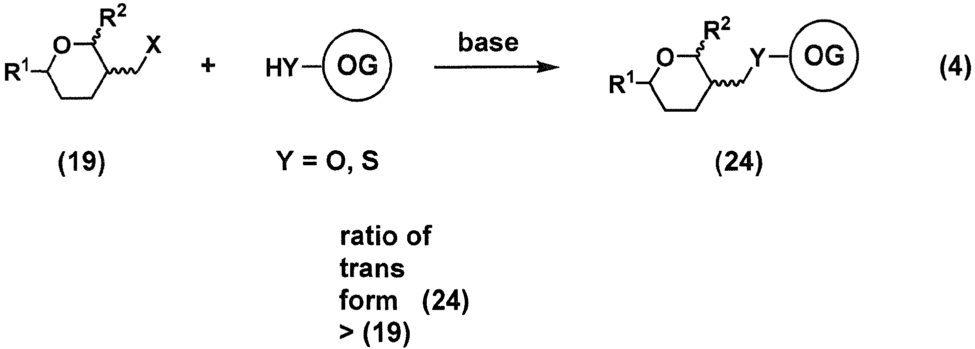

In formula (4), OG is an organic group.

The compound that is represented by formula (1) and is suitable for the liquid crystal compound can be synthesized by using compound (A), compound (B), compound (C), compound (20), compound (21), compound (22) or compound (23) according to the invention.

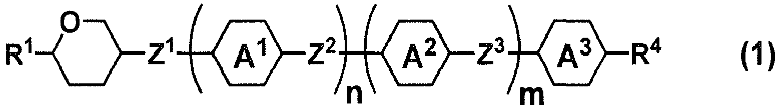

In formula (1),
R¹ is alkyl having 1 to 10 carbons;
R⁴ is hydrogen, cycloalkane having 3 to 8 carbons or alkyl having 1 to 10 carbons, and in the alkyl, at least one piece of -CH₂- may be replaced by -O-, and at least one piece of -CH₂CH₂- may be replaced by -CH=CH-;
ring A¹, ring A² and ring A³ are independently 1,4-cyclohexylene, 1,4-cyclohexenylene, 1,4-phenylene, 1, 4-phenylene in which at least one piece of hydrogen is replaced by halogen, naphthalene-2,5-diyl, naphthalene-2,5-diyl in which at least one piece of hydrogen is replaced by halogen, tetrahydro-2H-pyran-2,5-diyl, pyrimidine-2,5-diyl, pyridine-2,5-diyl or 1,3-dioxane-2,5-diyl;
Z¹ is -CH₂CH₂-, -CH=CH-, -CH₂O- or -COO-;
Z² and Z³ are independently a single bond, -CH₂CH₂-, -C=C-, -CH=CH-, -CF₂O-, -OCF₂-, -OCH₂-, -CH₂O-, -OCO-, -COO-, -CH₂CH₂CH₂O- or -OCH₂CH₂CH₂-; and
n and m are independently 0 or 1.

Specific preferred examples of R¹ include -CH₃, -C₂H₅, -C₃H₇, -C₄H₉ and -C₅H₁₁.

Specific preferred example of R⁴ include -CH₃, -C₂H₅, -C₃H₇, -C₄H9, -C₅H₁₁, -CH=CH₂, -CH=CHCH₃, -CH=CHC₂H₅, -CH=CHC₃H₇, - (CH₂)₂CH=CH₂, -(CH₂)₂CH=CHCH₃, -CH=CH(CH₂)₂CH=CH₂, (CH₂)₂CH=CH(CH₂)₂CH=CH₂, -OCH₃, -OC₂H₅, -OC₃H₇ and -OC₄H₉.

Specific preferred examples of ring A¹, ring A² or ring A³ include 1,4-cyclohexylene, 1,4-cyclohexenylene, 1,4-phenylene, 2-fluoro-1,4-phenylene and 2,3-difluoro-1,4-phenylene.

Specific preferred examples of Z¹ include -CH₂CH₂-, -CH=CH- and -CH₂O-.

Specific preferred examples of Z² or Z³ include a single bond, -CH₂CH₂-, -CH=CH-, -OCH₂- and -CH₂O-.

Then, 2-fluoro-1,4-phenylene means two divalent groups described below. In a chemical formula, fluorine may be leftward (L) or rightward (R). A same rule applies also to a divalent group derived from an asymmetrical ring such as tetrahydro-2H-pyran-2,5-diyl.

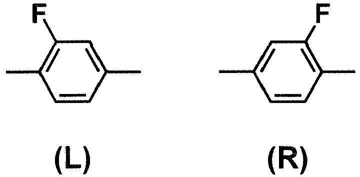

### Examples

The invention will be described in greater detail by way of Examples. However, the invention is not limited by the Examples.

### 1. Examples of compound (A), compound (B) and derivative

Compound (A), compound (B) and a derivative thereof were synthesized according to procedures described below. The synthesized compound was identified by methods such as an NMR analysis.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In the explanation of a nuclear magnetic resonance spectrum, s, d, t, q, quin, sex and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 30 m, bore 0.25 mm, film thickness 0.25 µm) made by Agilent Technologies, Inc. was used. Helium (1 mL/min) was used as a carrier gas. A temperature of a sample vaporizing chamber was set to 300°C and a temperature of a detector (FID) part was set to 300°C. A sample was dissolved in acetone and prepared to be a 1 wt% solution, and then 1 µL of the solution obtained was injected into the sample vaporizing chamber. As a recorder, GC Solution System made by Shimadzu Corporation or the like was used.

Gas chromatography mass spectrometry: For measurement, QP-2010 Ultra Series Gas Chromatograph Mass Spectrometer made by Shimadzu Corporation was used. As a column, a capillary column DB-1 (length 60 m, bore 0.25 mm, film thickness 0.25 µm) made by Agilent Technologies, Inc. was used. Helium (1 mL/min) was used as a carrier gas. For measurement, a temperature of a sample vaporization chamber was set to 300°C, a temperature of an ion source was set to 200°C, an ionization voltage was set to 70 eV, and an emission current was set to 150 µA. A sample was dissolved in acetone and prepared to be a 1 w% solution, and then 1 µL of the solution obtained was injected into the sample vaporizing chamber. As a recorder, GCMS Solution System made by Shimadzu Corporation was used.

HPLC analysis: For measurement, Prominence (LC-20 AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set at 254 nm. A sample was dissolved in acetonitrile and prepared to be a 0.1 w% solution, and then 1 µL of the solution was injected into a sample chamber. As a recorder, C-R7 Aplus made by Shimadzu Corporation was used.

### Example 1

### Synthesis of 2-propyl-3,4-dihydro-2H-pyran-5-carboxylic acid methyl ester

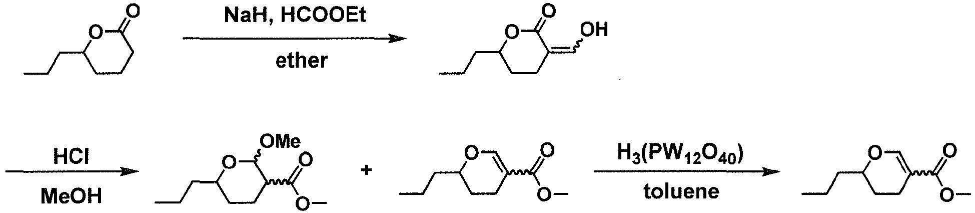

### First step:

Into an ether suspension of sodium hydride (26.14 g, 599.16 mmol), an ether solution of 6-propyltetrahydro-2H-pyran-2-one (71.00 g, 499.30 mmol) and ethyl formate (44.39 g, 599.16 mmol) was added dropwise under ice-cooling. The resulting reaction mixture was stirred at room temperature for 2 hours, and then refluxed under heating for 5 hours. The resulting mixture was poured into a 1 N hydrochloric acid aqueous solution, and subjected to extraction with ethyl acetate. Combined organic layers were washed with saturated brine, and dried over anhydrous magnesium sulfate. A solvent was distilled off to obtain 3-(hydroxymethylene)-6-propyltetrahydro-2H-pyran-2-one (84.99 g, yield: 99.0%).

### Second step:

Into a methanol solution of 3-(hydroxymethylene)-6-propyltetrahydro-2H-pyran-2-one (74.99 g, 440.58 mmol) obtained in the first step, concentrated hydrochloric acid (36.7 mL, 440.58 mmol) was added, and the resulting mixture was stirred at room temperature for 3 hours. The resulting reaction solution was poured into water, and subjected to extraction with ethyl acetate. Combined organic layers were washed with a saturated aqueous solution of sodium hydrogencarbonate, water and saturated brine, and dried over anhydrous magnesium sulfate. A solvent was distilled off, and the residue was purified by silica gel chromatography to obtain a mixture (78.86 g) of 2-methoxy-6-propyl-tetrahydro-2H-pyran-3-carboxylic acid methyl ester and 2-propyl-3,4-dihydro-2H-pyran-5-carboxylic acid methyl ester.

### Third step:

A mixture of (78.86 g) of 2-methoxy-6-propyl-tetrahydro-2H-pyran-3-carboxylic acid methyl ester and 2-propyl-3,4-dihydro-2H-pyran-5-carboxylic acid methyl ester obtained in the second step, and 12 Tungsto (VI) phosphoric acid n-hydrate (0.394 g, 0.14 mmol) was added to toluene, and the resulting mixture was refluxed for 3 hours under attaching a Dean-Stark condenser. The resulting reaction mixture was washed with a saturated aqueous solution of sodium hydrogencarbonate, water and saturated brine, and dried over anhydrous magnesium sulfate. A solvent is distilled off to obtain 2-propyl-3,4-dihydro-2H-pyran-5-carboxylic acid methyl ester (65.71 g, yield: 80.5% (a second step)).

### Example 2

### Synthesis of (2-propyl-3,4-dihydro-2H-pyran-5-yl)methanol

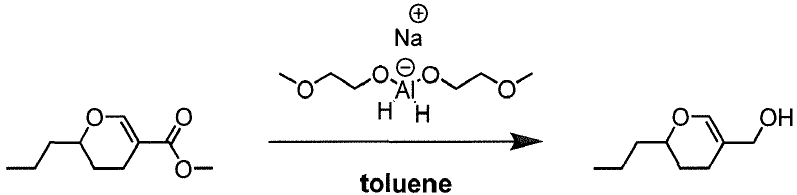

Into a toluene solution of sodium bis(2-methoxyethoxy)aluminum hydride (3.6 M toluene solution, 16.6 mL, 59.71 mmol), a toluene solution of 2-propyl-3,4-dihydro-2H-pyran-5-carboxylic acid methyl ester (10.00 g, 54.28 mmol) obtained in Example 1 was added dropwise under ice-cooling. The resulting reaction solution mixture was stirred at 40°C for 1 hour, and then poured into an aqueous solution of sodium potassium tartrate, and subjected to extraction with ethyl acetate. Combined organic layers were washed with water and saturated brine, dried over anhydrous magnesium sulfate, and a solvent was distilled off. The residue was purified by silica gel chromatography to obtain (2-propyl-3,4-dihydro-2H-pyran-5-yl)methanol (7.26 g, 46.47 mmol).

### Example 3

### Synthesis of trans-5-((4-ethoxy-2,3-difluorophenoxy)methyl)-2-propyltetr ahydro-2H-pyran

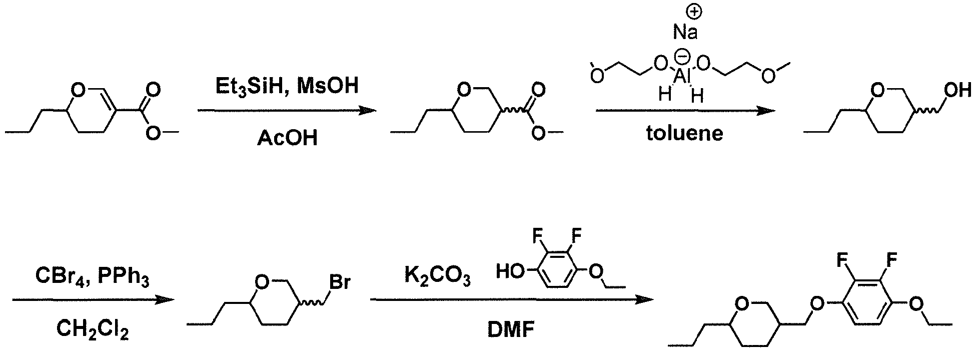

### First step:

Into an acetic acid solution of 2-propyl-3,4-dihydro-2H-pyran-5-carboxylic acid methyl ester (44.29 g, 240.40 mmol) obtained in Example 1 and triethylsilane (83.86 g, 721.20 mmol), methanesulfonic acid (69.31 g, 721.20 mmol) was added dropwise under ice-cooling. The resulting reaction solution was stirred at room temperature for 3 hours, and then poured into water, and subjected to extraction with ethyl acetate. Combined organic layers were washed with water and saturated brine, dried over anhydrous magnesium sulfate, and then a solvent was distilled off. The residue was purified by silica gel chromatography to obtain 6-propyl-tetrahydro-2H-pyran-3-carboxylic acid methyl ester (39.00 g, yield: 86.7%). A ratio of isomers in the product obtained was cis : trans = 17.4 : 82.6.

### Second step:

Into a toluene solution of sodium bis(2-methoxyethoxy)aluminum hydride (3.6 M toluene solution, 23.8 mL, 86.56 mmol), a toluene solution of 2-propyl-3,4-dihydro-2H-pyran-5-carboxylic acid methyl ester (14.66 g, 77.80 mmol) obtained in Example 1 was added dropwise under ice-cooling. The resulting reaction solution mixture was stirred at 40°C for 1 hour, and then poured into a 1 N hydrochloric acid aqueous solution, and subjected to extraction with ethyl acetate. Combined organic layers were washed with water and saturated brine, and dried over anhydrous magnesium sulfate. A solvent was distilled off to obtain (6-propyltetrahydro-2H-pyran-3-yl)methanol (10.00 g, yield: 80.2%). A ratio of isomers in the product obtained was cis : trans = 16.7 : 83.3.

### Third step:

Into a dichloromethane solution of (6-propyltetrahydro-2H-pyran-3-yl)methanol (10.00 g, 63.19 mmol) obtained in the second step and carbon tetrabromide (22.00 g, 66.35 mmol), triphenyl phosphine (17.40 g, 66.35 mmol) was added under ice-cooling. The resulting reaction mixture was stirred at room temperature for 1 hour, and then the resulting mixture was concentrated by an evaporator. The residue was purified by silica gel chromatography to obtain 5-(bromomethyl)-2-propyltetrahydro-2H-pyran (11.00 g, yield: 78.7%). A ratio of isomers in the product obtained was cis : trans = 8.8 : 91.2.

### Fourth step:

A mixture of 5-(bromomethyl)-2-propyltetrahydro-2H-pyran (11.00 g, 49.74 mmol) obtained in the third step, potassium carbonate (6.87 g, 49.74 mmol) and 4-ethoxy-2,3-difluorophenol (8.66 g, 49.74 mmol) was added to DMF, and the resulting mixture was stirred at 70°C for 5 hours. The resulting reaction mixture was poured into water, and subjected to extraction with toluene. Combined organic layers were washed with water and saturated brine, dried over anhydrous magnesium sulfate, and a solvent was distilled off. The residue was purified by silica gel chromatography and recrystallization to obtain trans-5-((4-ethoxy-2,3-difluorophenoxy)methyl)-2-propyltetr ahydro-2H-pyran (9.32 g, yield: 59.6%). A ratio of isomers in the crude product was cis : trans = 1.9 : 98.1.

¹H-NMR (CDCl₃; δ ppm): 6.63 - 6.57 (2H, m), 4.15 (1H, ddd, J = 11.2, 4.1, 2.2 Hz), 4.05 (2H, q, J = 7.0 Hz), 3.82, 3.75 (2H, ABqd, J = 9.5, 5.3, 7.1 Hz), 3.26 (1H, dd, J = 11.2, 11.2 Hz), 3.25 - 3.21 (1H, m), 2.15 - 2.06 (1H, m), 1.95 - 1.91 (1H, m), 1.71 - 1.67 (1H, m), 1.57 - 1.31 (6H, m), 1.41 (3H, t, J = 7.0 Hz), 0.92 (3H, t, J = 7.1 Hz).

### Example 4

### Synthesis of 6-pentyl-tetrahydro-2H-pyran-3-carboxylic acid methyl ester

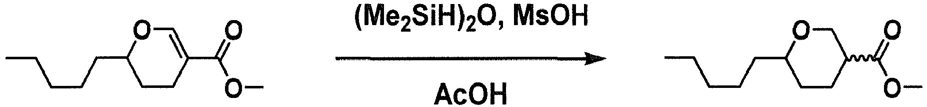

Into an acetic acid solution of 2-pentyl-3,4-dihydro-2H-pyran-5-carboxylic acid methyl ester (2.00 g, 9.42 mmol) synthesized according to the method described in Example 1 and 1,1,3,3-tetramethyldisiloxane (1.89 g, 28.26 mmol), methanesulfonic acid (2.72 g, 28.26 mmol) was added dropwise under ice-cooling. The resulting reaction solution was stirred at room temperature for 3 hours, and then poured into water, and subjected to extraction with ethyl acetate. Combined organic layers were washed with water and saturated brine, dried over anhydrous magnesium sulfate, and a solvent was distilled off. The residue was purified by silica gel chromatography to obtain 6-pentyl-tetrahydro-2H-pyran-3-carboxylic acid methyl ester (1.94g, yield: 96.0%). A ratio of isomers in the product obtained was cis : trans = 18.4 : 81.6.

### Example 5

### Synthesis of 5-((4-ethoxy-2,3-difluorophenoxy)methyl)-2-pentyltetrahydro -2H-pyran

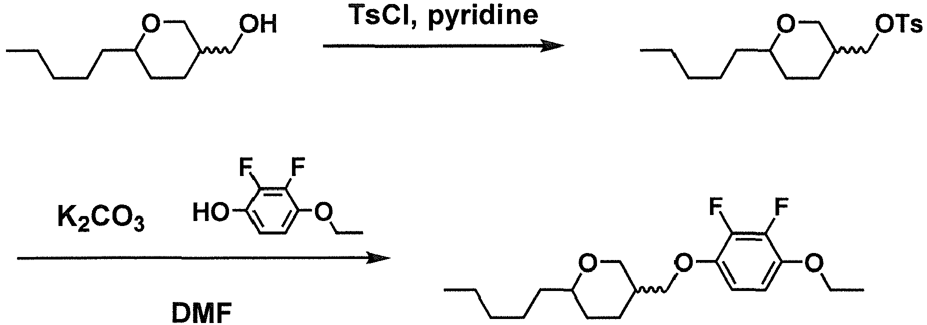

### First step:

Into a dichloromethane solution of (6-pentyltetrahydro-2H-pyran-3-yl)methanol (cis : trans = 11.1 : 88.9) (8.00 g, 42.94 mmol) synthesized according to a method in a manner similar to Example 2 and pyridine (3.74 g, 47.24 mmol), p-toluenesulfonyl chloride (8.60 g, 45.09 mmol) was added under ice-cooling. The resulting mixture was stirred overnight, and then poured into a 1 N hydrochloric acid. A dichloromethane layer was washed with water and saturated brine, dried over anhydrous magnesium sulfate, and a solvent was distilled off. The residue was used for the next step without purification. Formation of (6-pentyltetrahydro-2H-pyran-3-yl)methyl 4-methylbenzene sulfonate was confirmed by gas chromatography mass analysis.

### Second step:

A mixture of (6-pentyltetrahydro-2H-pyran-3-yl)methyl 4-methylbenzene sulfonate (14.62 g, 42.94 mmol) obtained in the first step, potassium carbonate (5.93 g, 42.94 mmol) and 4-ethoxy-2,3-difluoro phenol (7.47 g, 42.94 mmol) was added to DMF, and the resulting mixture was stirred at 70°C for 5 hours. The reaction mixture was poured into water, and subjected to extraction with toluene. Combined organic layers were washed with water and saturated brine, dried over anhydrous magnesium sulfate, and a solvent was distilled off. Formation of 5-((4-ethoxy-2,3-difluorophenoxy)methyl)-2-propyltetrahydro -2H-pyran was confirmed by gas chromatography mass analysis. A ratio of isomers in the crude product was cis : trans = 5.8 : 94.2.

¹H-NMR (CDCl₃; δ ppm) : 6.65 - 6.59 (2H, m), 4, 18 (1H, ddd, J = 11.2, 4.1, 2.2 Hz), 4.07 (2H, q, J = 7.0 Hz), 3.84, 3.78 (2H, ABqd, J = 9.5 Hz, 5.5, 7.1 Hz), 3.28 (1H, dd, J = 11.1, 11.1 Hz), 3.27 - 3.22 (1H, m), 2.17 - 2.09 (1H, m), 1. 97 - 1.93 (1H, m), 1.74 - 1.70 (1H, m), 1.58 - 1.51 (1H, m), 1.49 - 1.26 (9H, m), 1.44 (3H, t, J = 7.0Hz), 0.91 (3H, t, J = 7.3 Hz).

### Example 6

### Synthesis of trans-5-((4-ethoxy-2,3-difluorophenoxy)methyl)-2-pentyltetr ahydro-2H-pyran

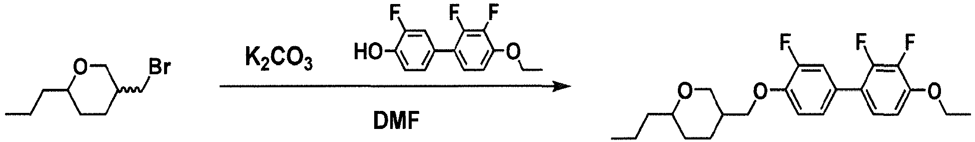

A mixture of 5-(bromomethyl)-2-propyltetrahydro-2H-pyran (cis : trans = 17.8 : 82.2) (5.00 g, 18.54 mmol), potassium carbonate (5.12 g, 37.08 mmol) and 4'-ethoxy-2',3,3'-trifluoro-[1,1'-biphenyl]-4-ol (5.22 g, 19.47 mmol) was added to DMF, and the resulting mixture was stirred at 70°C for 5 hours. The resulting reaction mixture was poured into water, and subjected to extraction with toluene. Combined organic layers were washed with water and saturated brine, dried over anhydrous magnesium sulfate, and a solvent was distilled off. The residue was purified by silica gel chromatography and recrystallization to obtain trans-5-((4'-ethoxy-2',3,3'-trifluoro-[1,1'-biphenyl]-4-yl) oxy)methyl-2-propyltetrahydro-2H-pyran (4.55 g, yield: 60.0%). A ratio of isomers in the crude product was cis : trans = 6.4 : 93.6.

¹H-NMR (CDCl₃; δ ppm) : 7.23 (1H, dt, J = 12.3, 1.4 Hz), 7.19 (1H, d, J = 8.7 Hz), 7.03 (1H, td, J = 8.6, 2.2 Hz), 6.97 (1H, t, J = 8.7 Hz), 6.77 (1H, dd, J = 8.3, 1.8 Hz), 4.18 (1H, ddd, J = 11.2, 4.2, 2.2 Hz), 4.15 (2H, q, J = 7.1 Hz), 3.90, 3.84 (2H, ABqd, J = 9.5, 5.4, 7.0 Hz), 3.28 (1H, dd, J = 11.5, 11.1 Hz), 3.28 - 3.23 (1H, m), 2.20 - 2.12 (1H, m), 1.99 - 1.96 (1H, m), 1.72 - 1.68 (1H, m), 1.55 - 1.30 (6H, m), 1.47 (3H, t, J = 7.0 Hz), 0.92 (3H, t, J = 7.3Hz).

### Example 7

### Synthesis of (E)-5-(2-(4-(2,3-difluoro-4-propylphenyl)cyclohexyl)vinyl)-2-propyltetrahydro-2H-pyran

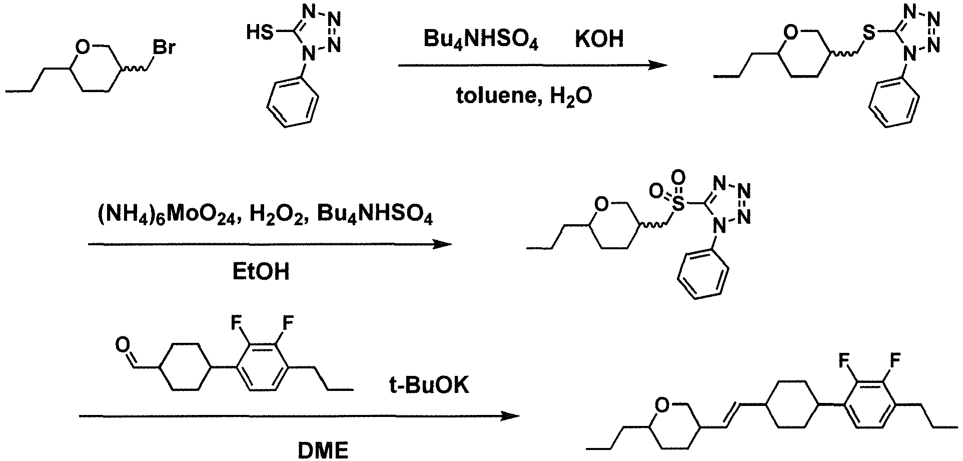

### First step:

A mixture of 5-(bromomethyl)-2-propyltetrahydro-2H-pyran (cis: trans = 17.8:82.2) (10.33 g, 46.71 mmol), 1-phenyl-1H-tetrazole-5-thiol (7.57 g, 42.47 mmol), tetrabutylammonium hydrogensulfate (1.44 g, 4.25 mmol) and potassium hydroxide (2.62 g, 46.71 mmol) was added to a toluene-water mixed solvent, and the resulting mixture was stirred at room temperature for 3 hours. A toluene layer was washed with water and saturated brine, dried over anhydrous magnesium sulfate, and a solvent was distilled off. The residue was purified by silica gel chromatography to obtain 1-phenyl-5-(((6-propyltetrahydro-2H-pyran-3-yl)methyl)thio) -1H-tetrazole (9.64 g, yield: 71.3%).

### Second step:

Into an ethanol solution of 1-phenyl-5-(((6-propyltetrahydro-2H-pyran-3-yl)methyl)thio) -1H-tetrazole (9.64 g, 30.27 mmol) obtained in the first step, tetrabutylammonium hydrogensulfate (1.03 g, 3.03 mmol) and ammonium molybdate (1.00 g, 1.51 mmol), 30% hydrogen peroxide water was added dropwise at 30°C or lower. The resulting mixture was stirred overnight, and then poured into water, and subjected to extraction with toluene. Combined organic layers were washed with water and saturated brine, dried over anhydrous magnesium sulfate, and a solvent was distilled off. The residue was purified by recrystallization to obtain 1-phenyl-5-(((6-propyltetrahydro-2H-pyran-3-yl)methyl)sulfo nyl)-1H-tetrazole (9.08 g, yield: 85.6%).

### Third step:

Into a dimethoxyethane solution of 1-phenyl-5-(((6-propyltetrahydro-2H-pyran-3-yl)methyl)sulfo nyl)-1H-tetrazole (7.59 g, 21.66 mmol) obtained in the second step and 4-(4-ethoxy-2,3-difluorophenyl)cyclohexane-1-carboaldehyde (7.57 g, 42.47 mmol), potassium t-butoxide was added at -65°C. The resulting reaction mixture was returned to room temperature, and then stirred at 60°C for 2 hours. The resulting reaction mixture was poured into water, and subjected to extraction with toluene. Combined organic layers were washed with water and saturated brine, dried over anhydrous magnesium sulfate, and a solvent was distilled off. The residue was purified by silica gel chromatography to obtain (E)-5-(2-(4-(2,3-difluoro-4-propylphenyl)cyclohexyl)vinyl)-2-propyltetrahydro-2H-pyran (1.40 g, yield: 19.7%).

¹H-NMR (CDCl₃; δ ppm) : 6.83 (1H, td, J = 8.9, 2.2 Hz), 6.66 (1H, ddd, J = 16.5, 8.1, 1.8 Hz), 5.44, 5.19 (2H, AXqd, J = 15. 8, 6.9 Hz), 4.09 (2H, q, J = 7.0 Hz), 3.86 (1H, ddd, J = 11.2, 4.4, 2.1 Hz), 3.21 - 3.16 (1H, m), 3.10 (1H, dd, J = 14.1, 11.2 Hz), 2.73 (1H, tt, J = 12.2, 3.2 Hz), 2.21 - 2.15 (1H, m), 1.98 - 1.90 (1H, m), 1.88 - 1.81 (5H, m), 1.65 - 1.63 (1H, m), 1.53 - 1.20 (10H, m), 1.43 (3H, t, J = 7.0 Hz), 0.91 (3H, t, J = 7.3 Hz).

### Industrial Applicability

A tetrahydro-2H-pyran derivative and an intermediate thereof can be provided further easily with a lower cost by using a production method of the invention in comparison with conventional arts. In particular, a trans-tetrahydro-2H-pyran-2,5-diyl derivative suitable for a liquid crystal compound can be provided.

## Claims

1. A production method for a tetrahydro-2H-pyran derivative, applying compound (A), compound (B) or compound (C) as an intermediate: 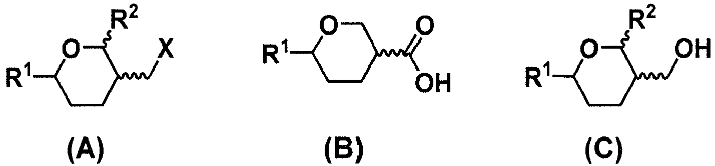 wherein, in compound (A), compound (B) and compound (C), R¹ is alkyl, alkenyl or alkoxy, R² is hydrogen or alkoxy, and X is a leaving group.

2. The production method for the tetrahydro-2H-pyran according to claim 1, wherein, in compound (A), compound (B) or compound (C), R¹ is alkyl, X is nonafluorosulfonate, trifluoromethanesulfonate, fluorosulfonate, tosilate, mesilate, iodine, bromine or chlorine, and R² is hydrogen.

3. The production method for the tetrahydro-2H-pyran derivative according to claim 1 or 2, wherein compound (A), compound (B) or compound (C) that may be substituted and derived from 5-valerolactone is applied as an intermediate.

4. The production method for the tetrahydro-2H-pyran derivative according to any one of claims 1 to 3, wherein a compound represented by formula (1) is an objective substance: 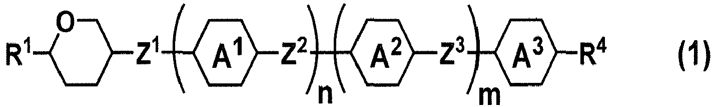 wherein, in formula (1),
R¹ is alkyl having 1 to 10 carbons;
R⁴ is hydrogen, cycloalkane having 3 to 8 carbons or alkyl having 1 to 10 carbons, and in the alkyl, at least one piece of -CH₂- may be replaced by -O-, and at least one piece of -CH₂CH₂- may be replaced by -CH=CH-;
ring A¹, ring A² and ring A³ are independently 1,4-cyclohexylene, 1,4-cyclohexenylene, 1,4-phenylene, 1,4-phenylene in which at least one piece of hydrogen is replaced by halogen, naphthalene-2,5-diyl, naphthalene-2,5-diyl in which at least one piece of hydrogen is replaced by halogen, tetrahydro-2H-pyran-2,5-diyl, pyrimidine-2,5-diyl, pyridine-2,5-diyl or 1,3-dioxane-2,5-diyl;
Z¹ is -CH₂CH₂-, -CH=CH-, -CH₂O- or -COO-;
Z² and Z³ are independently a single bond, -CH₂CH₂-, -C=C-, -CH=CH- -CF₂O-, -OCF₂-, -OCH₂-, -CH₂O-, -OCO-, -COO-, -CH₂CH₂CH₂O- or -OCH₂CH₂CH₂-; and
n and m are independently 0 or 1.

5. The production method for the tetrahydro-2H-pyran derivative according to any one of claims 1 to 4, comprising a step of etherification reaction.

6. A compound represented by formula (2): 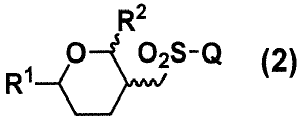 wherein, in formula (2), R¹ is alkyl, alkenyl or alkoxy, and R² is hydrogen or alkoxy; and
Q is any one of structures described below:

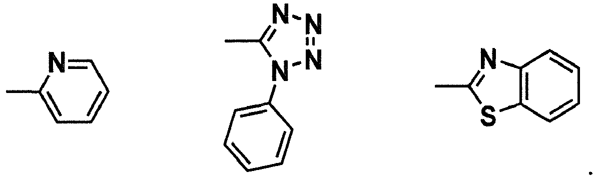

7. Utilization of the compound that is represented by formula (1) and produced by the production method according to claim 4 or 5 as a component of a liquid crystal medium.
